# EUROPEAN PATENT SPECIFICATION

(11) **EP 0 704 224 B1**
(45) Date of publication and mention of the grant of the patent: **27.02.2002**
(21) Application number: 95203214.2
(22) Date of filing: 28.03.1991
(51) Int. Cl.: A61M 1/34, A61M 1/36

(54) **Method of monitoring fluid flow**
Verfahren zum Überwachen eines Flüssigkeitsstromes
Méthode de contrôle d'un écoulement de fluide

(30) Priority: 30.03.1990 US 502395
(43) Date of publication of application: 03.04.1996
(62) Divisional of application: 91907885.7
(73) Proprietor: BAXTER INTERNATIONAL INC., Deerfield, IL 60015-4633 (US)
(72) Inventor: Ford, Michael G., Orange, California 92669 (US); Prince, Paul R., San Juan Capistrano, California 92675 (US)
(74) Representative: MacGregor, Gordon

(56) References cited:
- EP-A- 0 128 683
- MERZ H ET AL: "EIN STERILES, GEREGELTES, PULSATIONSARMES PRAZISIONS-DOSIERSYSTEM FUER DIE BIOMEDIZINTECHNIK" 1 January 1990 , BIOMEDIZINISCHE TECHNIK, VOL. 35, NR. 1 / 02, PAGE(S) 5 - 9 XP000133835 * page 1, column 1, line 14 - page 6, column 1, line 4 * * figure 1 *
- PROCEEDINGS OF MELECON, vol. 1, 8 - 10 October 1985, MADRID, pages 117-119, XP002009057 M METWALLY : "microprocessor based hemofiltration system for renal insufficiency"

## Description

### 1. Field of the Invention

This invention relates to a method of monitoring fluid flow, especially for metering flow from multiple liquid sources, for example for plasmapheresis blood flow control.

### 2. Discussion of the Prior Art

In fluid flow systems the need arises for controlling sequential or simultaneous influx from multiple fluid sources, especially those of limited capacity. In some cases it is desirable to monitor the total influx from such fluid sources so as to match the amount of fluid withdrawn from the system at another point or in another operation. For instance, plasmapheresis systems are known which require the withdrawal from and subsequent reinfusion of bodily fluids to a living subject in known or fixed amounts. The subject is usually a human or animal, but might also be a cadaver.

In the case of a plasmapheresis system, whole blood is extracted from the subject, plasma is separated from the whole blood, and an extraction product containing a higher concentration of blood cells than the whole blood is reinfused into the subject while the separated plasma is retained and used for desired purposes, treated to alleviate an undesirable condition, or discarded as unfit for further use. To compensate for plasma removed from the patient, a selected volume of replacement fluid is infused into the patient to replace the volume of plasma separated from the whole blood. The use of replacement fluids often has a therapeutic aim. Many undesirable components of blood, including diseased cells, antigens, and the like, are held in suspension therein. During therapeutic plasma exchange, these undesirable components are separated from the blood and removed with the plasma fraction, leaving the blood cleansed and healthier. The replacement fluids used during therapeutic plasma exchange can comprise a variety of fluids, such as saline, thawed plasma, and various therapeutic fluids, depending upon the desires of the treating physician. In such a case it is often desirable to control the amount and rate of each of the replacement fluids administered to the patient.

Determining the exact amount of any given replacement fluid administered to the patient during therapeutic plasma exchange is complicated by the use in modern plasmapheresis machines of disposable plastic tubing harness sets. Although manufactured to specifications as exact as possible, the flow characteristics of each plastic harness set differ somewhat from those of all others.

Therefore, despite all efforts to calibrate the peristaltic pumps with which the harness sets are used to provide sterile, non- invasive therapy, the pumps deliver at slightly different rates dependent upon the individual flow characteristics of the harness set used. The need exists, therefore, for a method of monitoring and controlling with increased precision the amount of each replacement fluid used during therapy.

Yet another problem inherent in monitoring the weight of process fluids comes from inaccuracies introduced due to motion of the fluids being monitored. For instance, the motion of replacement fluids flowing from a weighing bag is sufficient to affect the accuracy of delicate balances used in weighing by mechanical means. The need exists, therefore, for a way to isolate the fluids weighed from the effects of mass transport.

To optimize use of processing equipment and support personnel while minimizing inconvenience and discomfort to the patient, it is also desirable to reinfuse bodily fluids as rapidly and safely as possible. However, replacement fluids, usually saline, are commonly prepackaged in sterile containers of predetermined volume, necessitating that more than one container of replacement fluid be infused into the patient to replace the plasma removed during a typical session of plasmapheresis. If the containers of saline are fed sequentially into the replacement line for reinfusion, an attendant must monitor the flow of replacement fluid, and manually switch flow from one emptying bag to the next full bag before air enters the replacement line from an empty bag. In some cases the attendant must momentarily stop the flow of replacement fluid to switch flow to a new bag.

If air is inadvertently allowed to enter the infusion line from an empty bag, the machine must be stopped so that air can be removed from the flow line before it enters the patient's blood. In some cases, the entire plasmapheresis session must be scrapped and begun anew. Frequently, if a large amount of blood has been withdrawn, the patient cannot continue with a new session until sufficient time has elapsed to recuperate from the effects of the first session. Time and effort are lost while the patient is exposed to an unnecessary health risk.

The problem of replacement fluid sources going dry is compounded when multiple fluids at different rates and from separate sources are infused either sequentially or simultaneously through the single venipuncture needle. For instance, typically the reinfusion mixture contains concentrated red blood cells and sufficient anticoagulant to prevent coagulation of the red cells. At the same time, a replacement fluid, usually saline, is introduced at a rate sufficient to substitute for the plasma removed. Under certain circumstances it may be desirable to add another fluid to the mixture, such as albumin, frozen plasma, a medicament, or the like. The need exists, therefore, for a method and apparatus adapted to prevent multiple reinfusion fluid sources from going dry while monitoring the total amounts and/or relative infusion rates of the multiple reinfusion sources.

The art has long sought apparatus and equipment useful for monitoring the flow of liquid systems. It is known to monitor the flow of liquids into a fluid flow system by use of various devices. For example, in U.S. Patent 4,655,742 and European patent application No. 232,263, optical detectors are used to determine when a container of fluids is full. Automatic weighing can also be effected by means of an electrical load cell that actuates an electronic device to squeeze off a tube and thereby prevent further filling of a container as disclosed in German patent No. DE 3 739 240.

Weight scales are also known for measuring the flow of blood into or out of a container. For instance, German patent No. DE 3 737 304 discloses a weighing pan connected to a pivot such that at a certain weight of blood in a bag resting on the weighing pan, a compression valve is activated to choke the flow of blood into the bag or interrupt it intermittently. In this way, unnecessary load upon the blood donor's circulatory system and heart are avoided. In addition, European Patent Application EP 87907352, filed October 11, 1987, discloses a blood separation device that holds and weighs at least two separation bags that communicate with the blood bag by means of a tube. Alternatively, as disclosed in Spanish Patent No. 8 801 535, a mechanical balancing system monitoring the difference between a total instantaneous weight and an instantaneous equilibrium force can be used to regulate the flow of substitute fluid in a blood filtration device.

However, none of these devices warns the attendant when the replacement fluid source is going to run dry, provides a means for switching to an auxiliary source without temporarily stopping flow of replacement fluid into the reinfusion mixture, and/or meters the total amount of replacement fluid used from multiple sources. Thus, the need exists for new and better methods and apparatus for monitoring fluid flow systems, especially in plasmapheresis devices used for reinfusing replacement fluids to compensate for plasma removed and not reinfused.

DE-C-3637771 discloses a method of infusion in which fluid from a source is intermittently flowed into a measuring chamber and from the measuring chamber to a flow system. The flow from the source is controlled by means of an optical sensor which opens flow to the measuring chamber when a lower level is reached and closes flow to the chamber when an upper level is reached.

The precharacterising part of Claim 1 is based on this disclosure and the characterising parts of the claim set out the distinguishing features of the invention.

Biomed. Technik 35: 1990; p5-9 discloses metering of small amounts of solution by a peristaltic pump using a weighing device to measure quantities of solution between upper and lower limits.

The present invention uses a weighing bag in which the fluid is weighed. Fluid is flowed into the bag from a fluid source when a lower working weight limit is reached and stopped when a higher working weight is reached. A warning alarm is triggered when the weight of fluid in the weighing bag drops below a warning amount, below the lower working limit, and a replacement fluid source is connected to the weighing bag in response to the warning alarm, so that fluid flows from the replacement fluid source into the weighing bag until the weight of fluid in the weighing bag is raised above the warning amount while fluid continues to flow from the weighing bag.

The fluid source may include any number of containers. Preferably the containers are held by a holder so that fluid from the containers flows by gravity into the weighing bag. Replacement fluids are metered into the flow system from the weighing bag. The capacity of the weighing bag is sufficient to accommodate this function.

The weighing bag is large enough to allow continuous metering therefrom of replacement fluids after a warning alarm has been initiated and while the attendant replenishes the diminishing supply of fluid therein. Thus, the operation of a plasmapheresis machine need not be halted while the weighing bag is being replenished.

Fluids held in the weighing bag are metered therefrom under the operation of a control means equipped to initiate an audible or visual alarm signal when the weight of fluid in the weighing bag drops below a first higher warning level and to shut down the entire plasmapheresis operation or just the flow of replacement fluid into the system when the weight of fluid in the weighing bag drops below a second fail safe level. Successive batches of one of more fluids can be metered through the weighing bag while by these means the weighing bag is prevented from becoming empty, and air is thereby prevented from entering the flow line leading therefrom.

The cumulative amount of fluids metered through the weighing bag can be determined with great accuracy by two different methods. By the first method, flow from the weighing bag is temporarily halted when successive batches of fluids are flowed into the weighing bag and the sum total of the fluids passed through the bag is found by adding together the weights of successive batches of fluid passed into the weighing bag before flow therefrom is reinitiated.

The second method is used when continuous flow from the weighing bag is desired. By this method the cumulative amount of fluid passed through the weighing bag is determined by metering continuous flow from the weighing bag through a calibrated pump. The latter method of calculating continuous fluid flow is complicated when fluids are metered through the weighing bag by means of non-invasive peristaltic pumps and the flow lines comprise disposable plastic tubings with unique flow characteristics, as are typically used in plasmapheresis. In such cases, an absolute calibration of pump performance for the replacement fluid pump can be obtained, before or at any time during the plasmapheresis operation, using the multiple fluid flow isolation, control and alarm system herein. To calibrate the fluid replacement pump, the times required at different flow rates for a given weight of fluid of known density to pass out of the weighing bag by means of the pump and plastic set is observed. The weighing bag is allowed to fill to its maximum working weight and the clamp on the input line to the weighing bag is then closed. Thus the change in weight of fluid in the weighing bag is due solely to the operation of the pump. Using this data, the weight of replacement fluid pumped from the weighing bag at a given pump flow rate setting during any period of time can then be calculated from the time of continuous operation of the pump.

In the preferred embodiment, during normal operations the weight of fluid in the weighing bag cycles between upper and lower working level limits preselected for convenience, for example between an upper limit of 200 grams and a lower working limit of 100 grams. At the start of operations, the weighing bag is filled to the upper limit by opening the clamp on the input line and allowing an influx of fluid therein. Then the clamp is closed, the fluid pump is started, and the weight of fluid in the weighing bag continuously decreases down to the lower working limit, at which point the clamp on the input line is opened and fluid again flows into the weighing bag until the upper limit is reached, at which point the clamp again closes. In this embodiment of the invention, the pump can be automatically recalibrated each time the weight of fluid in the weighing bag moves from the upper to the lower working limit. The amount of fluid pumped during the next following filling period (while the weight of fluid moves from the lower to the upper working limit) is then calculated upon the basis of the immediately preceding pump calibration. In this way, the pump can be continually recalibrated to compensate for changes in the resiliency of the tubing set caused by temperature change, and the like.

The multiple fluid flow system herein provides an automatic warning alarm when the weight of fluid in the weighing bag drops below a certain predetermined warning level selected to be below the lower working limit. The attendant is given sufficient warning before the weighing bag empties that the supply of replacement fluids in the weighing bag can be replenished, manually if necessary, without shutting down the flow of fluids through the system. If the weight of the weighing bag continues to diminish, for instance, because the supply of fluid therein has not been replenished, at a second, lower predetermined fail safe weight of fluid the automatic fail-safe feature of the system shuts down all operations or stops the flow of the replacement fluids.

The flow control and alarm system herein also provides an automatic self-checking feature to eliminate the possible hazards inherent in automatically controlled operations. This feature automatically generates an error signal when, according to the most recent pump calibration, the pump has been running long enough to reduce the weight of the weighing bag from the upper working level to below the lower working level but the actual weight of fluid in the weighing bag has not decreased accordingly.

In the preferred embodiment, the multiple fluid flow isolation, control and alarm system of the invention is adapted to continuously meter known, exact amounts of replacement fluid, into a disposable plastic flow set of the type used with a typical plasmapheresis machine. The replacement fluid, usually several aliquots of saline from pre-packaged, sterile containers, usually holding about 500 to 1000 milliliters of fluid each, is added without allowing air to enter the flow set and mix with the blood to be returned to the patient. Other fluids can also be added either mixed with the saline or separately.

In addition to the multiple fluid flow isolation, control and alarm system described in detail herein, the flow set, sometimes known as a "harness set", includes a blood separator to separate out and collect a blood component while returning to the donor or patient the balance of the blood, along with sufficient replacement fluid to compensate for the component removed. When blood withdrawal and fluid reinfusion is cyclic, a single attachment means to the patient is used, such as a single phlebotomy needle. On the other hand, when both operations proceed simultaneously, the flow set includes two means of attachment to the donor or patient, such as separate withdrawal and reinfusion phlebotomy needles.

### Brief Description of the Drawings

A better understanding of the invention can be had from a consideration of the following detailed description, taken in conjunction with the accompanying drawings in which:
Fig. 1 is a schematic and block diagram representation of a fluid flow path for a plasmapheresis system using a multiple fluid source isolation, metering, and alarm system in accordance with the invention;

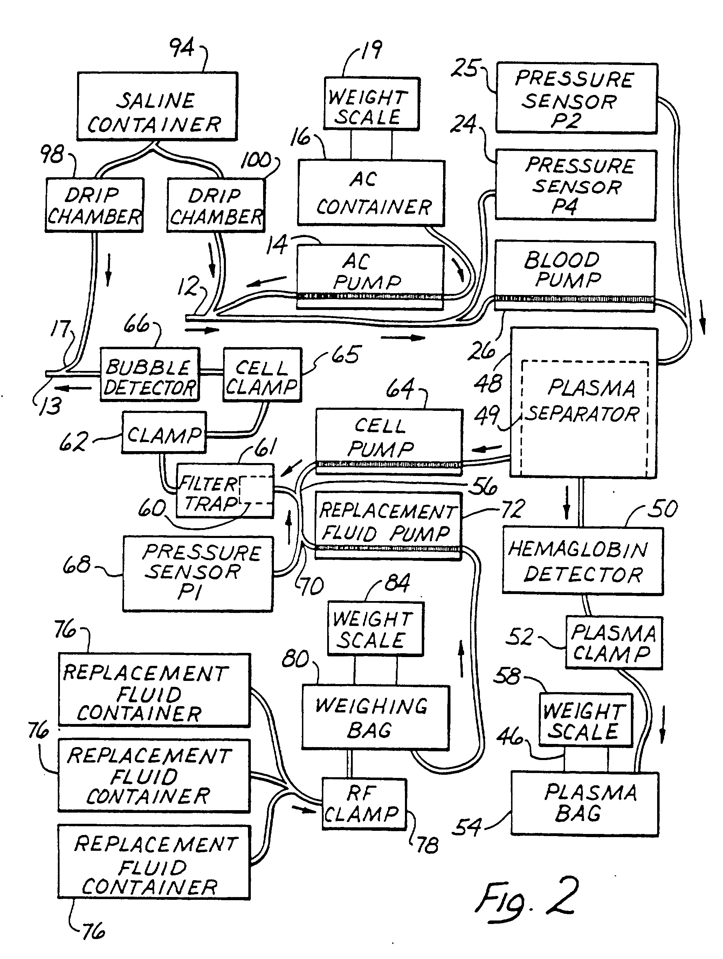

Fig. 2 is a functional block diagram representation of a plasmapheresis system incorporating a multiple fluid source isolation, metering, and alarm system in accordance with the invention;
Fig. 3 is a schematic and block diagram representation of a fluid flow path for a plasmapheresis system using a multiple fluid source isolation, metering, and alarm system in accordance with the invention wherein multiple fluid sources are connected to the weighing bag.

### Detailed Description of the Preferred Embodiment

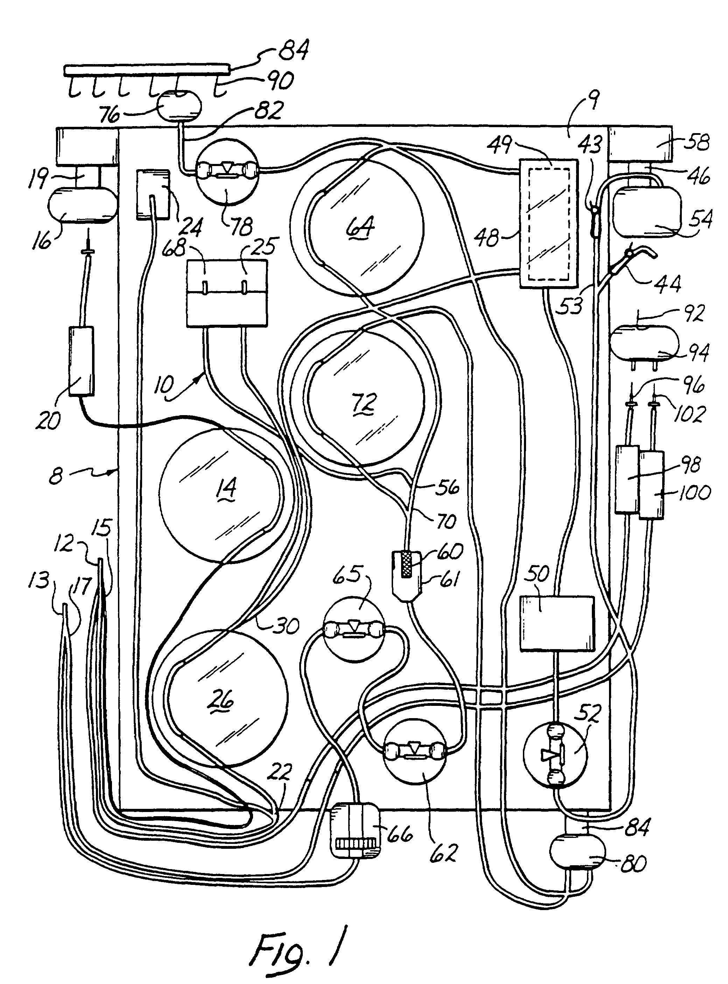

Referring now to Fig. 1, a conventional plasmapheresis instrument is generally designated 8 and includes various pumps, clamps, detectors, monitoring systems, indicators, and the like, not all of which are described in the present application or necessary to an understanding of the present invention. Therefore, only those parts of the instrument which are applicable for an understanding of the present invention will be described.

Instrument 8, on its face 9, includes four peristaltic pumps which are individually driven and under the control of a computerized control system. A disposable harness set, according to the present invention, is applied to the instrument and to the donor such that blood collection, separation and infusion of packed cells with replacement fluid can be provided. Flow path 10 of the disposable harness set will now be described.

Flow path 10 provides a noninvasive, sterile plasmapheresis flow path for a dual needle plasmapheresis system utilizing a multiple fluid flow isolation, control and alarm system in accordance with the invention. Intravenous connection of flow path 10 to a subject is provided by dual bodily fluid flow channel connections such as phlebotomy needles 12 and 13 which are suitable for insertion into the veins of a living (or deceased) subject to provide bidirectional communication of blood and other fluids between the subject and flow path 10 of the plasmapheresis system.

The flow path branches immediately adjacent withdrawal needle 12 at branch point 15 with one branch extending through a noninvasive peristaltic anticoagulant pump 14 and drip chamber 20 to an anticoagulant container 16 held by holder 19. During operation, anticoagulant pump 14 operates to supply and mix a small percentage of anticoagulant with the blood as it is being extracted to prevent activation of clotting mechanisms that would cause the blood to cling to tubing sidewalls as it passes through flow path 10. By mixing the anticoagulant with the whole blood at needle 12, the two fluids become fully mixed during withdrawal and less anticoagulant is required. This desirable result helps minimize the amount of anticoagulant introduced into the blood.

The other branch of blood flow path 10 leaving withdrawal needle 12 extends to another branch point 22. Optionally, from branch point 22 one branch extends to a pressure sensor 24 coupled to sense fluid pressure on the subject side of a blood pump 26. The pressure sensor 24 includes a disposable filter coupling the sensor to a pressure sensor tube (not shown) so as to maintain a noninvaded sterile atmosphere within the flow path 10. The second branch from branch point 22 extends through the noninvasive, peristaltic blood pump 26 to branch point 30. From branch point 30 one branch leads to pressure sensor 25, which also includes a disposable filter coupling to maintain sterility. Pressure sensor 25 detects pressure across filter 49 in separator 48. Another flow path from branch point 30 extends to the bottom of plasma separator 48, which encloses filter 49.

While the exact nature of the plasma separator 48 is not material to the present invention and can be fully conventional if desired, a highly advantageous plasma separator is a centrifugal filter type of separator as illustrated in U.S. 5194145. For this type of separator the end product plasma output is coupled through a hemoglobin detector 50 and a plasma clamp 52 through branch point 53 and clamp means 43, such as a roller clamp, to a plasma container 54, which is maintained at atmospheric pressure. The plasma container 54 is suspended from a hanger means 46 of a weight scale 58, which provides feedback to the plasmapheresis system of the amount of plasma within container 54. Another open ended flow path from branch point 53 extends through clamp means 44, such as a roller clamp, for attaching an auxiliary plasma container (not shown) to flow path 10.

A cell pump 64 coupled with an outlet of plasma separator 48 controls the reinfusion flow of high hematocrit blood from plasma separator 48 through a flow path extending through branch points 56, and 70, filter 60 and flexible bubble trap 61, blood clamps 62 and 65, air detector 66, branch point 17, and reinfusion needle 13. From branch point 56 another flow path extends to pressure sensor 68. Since plasma removed from separator 48 is maintained at atmospheric pressure plus a small adjustment for vertical height differences, the difference between pressure sensors 68 and plasma container 54 provides an indication of pressure across filter 49 within plasma separator 48. This pressure indication can be useful in monitoring and controlling the operation of plasma separator 48.

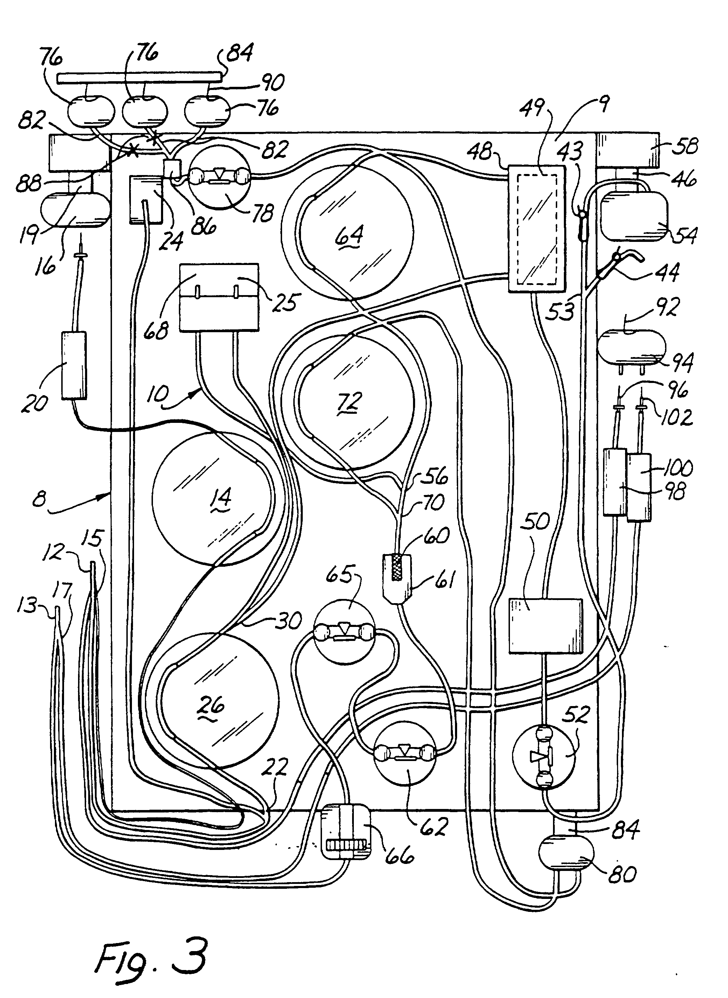

From branch point 70, another flow path extends through replacement fluid pump 72 to replacement fluid weighing bag 80. Weighing bag 80 is suspended from scale means 84, and another flow path extends from weighing bag 80 through clamp 78 and a connector 82 to one of a plurality of fluid containers 76. In an alternative embodiment, as shown in Fig. 3, from clamp 78 the flow path extends to drip chamber 86 at the exit from which it branches into a plurality of flow paths extending to a plurality of connectors, such as a plastic spikes, and individual fluid containers 76. A clamp means 88 is located along each reinfusion line 82 for independently controlling the flow of fluid therethrough to weighing bag 80. In drip chamber 86 fluids from any or all of containers 76 are received and mixed. Usually at least one of fluid containers 76 is filled with saline. Others of the fluid containers 76 can also be filled with supplementary bags of saline. In both embodiments shown in Figs. 1 and 3, fluid containers 76 are suspended or supportably attached by attachment means 90 to replacement fluid holder 84.

Returning now to Figure 1, from branch point 15 a third branch of the flow line extends through drip chamber 98 and connector 96 to saline bag 94 suspended from attachment means 92. From branch point 17 near reinfusion needle 13 a second branch of the flow path extends through drip chamber 100 and connector 102 to saline bag 94. Before and after the plasmapheresis cycle, saline from fluid container 94 can be passed through needles 12 and/or 13 to prime or cleanse the needle and thereby prevent clogging due to coagulation of blood therein. This flow path enables the separator to be primed with a small amount of saline prior to initial use and to be cleansed with saline after final use. If for any reason the plasmapheresis cycle is temporarily halted, saline can be dripped through needles 12 and 13 to keep them open until operations are reinitiated. The saline drip preferably can be initiated manually by the attendant opening control means (not shown), such as a roller clamp. Alternatively, the computerized control means can initiate the saline drip therethrough whenever the fail safe mechanism shuts down operations, as further described hereinbelow.

During normal operations the weight of fluid in the weighing bag cycles between upper and lower working level limits preselected for convenience, for example between an upper limit of 200 grams and a lower working limit of 100 grams. To initiate the cycle at the start of operations, after the system has been primed and the pump 72 has been initially calibrated, the weighing bag 80 is filled to the upper limit by opening the clamp 78 on the input line and allowing an influx of fluid therein. Then the clamp 78 is closed, pump 72 is started, and the weight of fluid in the weighing bag continuously decreases down to the lower working limit, at which point the clamp on the input line is opened and fluid again flows into the weighing bag until the upper limit is reached, at which point the clamp again closes. In this embodiment of the invention, the pump can be automatically recalibrated each time the weight of fluid in the weighing bag moves from the upper to the lower working limit. The amount of fluid pumped during the next following filling period (while the weight of fluid moves from the lower to the upper working limit) is then calculated upon the basis of the immediately preceding pump calibration. In this way, the pump can be continually recalibrated to compensate for changes in the resiliency of the tubing set caused by temperature change, and the like.

During plasmapheresis, using the apparatus and method of this invention, blood from the patient is withdrawn through needle 12, and sent through separator 48 for removal of plasma therefrom. Replacement fluids are simultaneously withdrawn from weighing bag 80 via pump 72, mixed with red cell concentrate at branch point 70 (pumped via pump 64 from the separator 48 outlet), and reinfused to the patient via needle 13. When used for plasma replacement therapy, plasma usually is considered waste and discarded; however, the plasma can also be retained and given therapeutic treatment by known means. Although one skilled in the art will appreciate that the apparatus could be modified to perform cyclic withdrawal and reinfusion functions, preferably withdrawal, separation and reinfusion proceed simultaneously. In either the cyclic or sequential modes of operation, the reinfusion mixture returned to the patient via reinfusion needle 13 comprises concentrated cells of increased hematocrit and anticoagulant recovered from the separator 48 and sufficient replacement fluid from individual replacement bag(s) 76 to replace in any proportion the amount of plasma collected in weighed plasma bag 54 as directed by the treating physician.

Attached to scale means 84 is an alarm means, not shown, for providing a visual or audible signal whenever during plasmapheresis the amount of fluid contained in weighing bag 80 drops below a preselected relatively low warning amount, e.g. from 65g to 85g; or preferably from 75 to 85 g. In addition, scale means 84 is provided with an automatic fail safe means (not shown) for shutting down the plasmapheresis operation whenever the amount of fluid in weighing bag 80 drops below a second, lower preselected fail safe amount. The difference between the warning amount and the fail safe amount is any convenient amount selected to allow the attendant sufficient time to add additional fluids to weighing bag 80 while pump 72 continues to run before it empties to the fail safe amount. The exact amount of the difference between the warning and fail safe amounts, of course, will usually differ depending upon the rate of pump 72, but may be 15g, or less.

The control and alarm system herein also provides an automatic self-checking feature to eliminate the possible hazards inherent in automatically controlled operations. The control means automatically generates an audible or visual error signal when pump 72 has been running with clamp means 78 closed long enough to deplete the weight of fluid in the weighing bag some preselected amount, preferably from the maximum working weight at initiation of operations, but the actual weight of fluid in the weighing bag has not dropped a corresponding amount.

In use, the harness set comprising flow path 10 is applied to face 9 of instrument 8 as illustrated in Figure 1. Separator 48 is placed into a motor mount (not shown) and flow path 10 is threaded into the anticoagulant pump 14, blood pump 26, cell pump 64, and replacement fluid pump 72, as well as clamps 78, 65, 62, and 52. Weighing bag 80 is hung from scale 84 and plasma bag 46 is hung from scale 58. Replacement fluid bags 76 are hung from attachment means 90 and at least one of them is attached to flow path 10 using a connector 82. Saline bag 94 is hung from attachment means 92 and attached to flow path 10 via connectors 96 and 102.

In operation, various set-up and safety procedures are followed and the attachment means 12 and 13 are phlebotomy needles applied to the patient. To utilize the multiple fluid flow isolation, control and alarm system features of the invention, clamp 78 is opened and a known quantity of replacement fluid from one or more of the replacement fluid bags 76 is allowed to circulate, for example by gravity flow, into replacement fluid weighing bag 80. Clamp 78 is then closed. The amount of fluid circulated into weighing bag 80 is sufficient to allow plasmapheresis operations to proceed at least until an additional isolated aliquot or batch of replacement fluids can be circulated into weighing bag 80 by repeating the above steps, usually at least 100 grams or more. Preferably, however, no more than between about 100 and 200 grams at a time are circulated into weighing bag 80 to facilitate switching from one replacement fluid type to another without emptying weighing bag 80. Optionally several of bags 76 can contain the same type of fluid and connector 82 can be moved from one to the next to empty several of bags 76 into weighing bag 80 in a single batch.

Plasmapheresis is initiated by opening clamps 52, 62 and 65 and starting pumps 26, 14, 64 and 72. Blood from the patient mixed with anticoagulant in proportions controlled by the relative rates of pumps 14 and 26 flows to separator 48 wherein plasma is separated and circulated to plasma bag 54. Simultaneously, concentrated cells recovered from separator 48 are circulated by pump 64 to branch point 70 where they are joined by a flow of replacement fluid from weighing bag 80 at a rate controlled by pump 72. The replacement fluid is usually delivered in some predetermined ratio to the amount of plasma being removed to plasma bag 54. When the amount of fluid in weighing bag 80 drops below the preselected warning amount, the alarm attached to weighing bag 80 is triggered to produce either an audible or visible alarm.

The alarm means and fail safe means attached to weighing means 84 can utilize known methods of triggering mechanical alarm. Preferably, however, the alarm and fail safe means are incorporated within a programmed digital processor that controls the operation of the plasmapheresis machine using known principles.

The warning alarm alerts the attendant that replacement fluid weighing bag 80 is approaching empty. In response to the warning alarm, the attendant can replenish the replacement fluid in bag 80 before it runs dry by either of two methods. By the first, less preferred method the attendant stops either pump 72 or all of pumps 26, 14, 64 and 72, checks to see that one of the replacement fluid bag(s) 76 contains at least an aliquot of fluid, and opens the clamp 78 on the bag 76 to allow fluids therefrom to empty into weighing bag 80 until the weight of replacement fluid in weighing bag 80 is increased above the warning level and the pump or pumps are all restarted. By the second, more preferred method the pumps are not stopped and the contents of bag 80 continue to empty while the auxiliary fluid replacement bags 76 are positioned by the attendant and emptied as described above into bag 80.

If the amount of replacement fluid in weighing bag 80 is not replenished before the weight of fluid therein drops to the fail safe amount, the automatic fail safe means associated with weighing means 84 shuts down operations by stopping either pump 72 alone or pumps 14, 26, 64, and 72 and closing clamps 52, 62 and 64, and generates a fail safe alarm message for the attendant. Once the control means has shut down operations, the control means stops or removes the fail safe alarm and operations of the system can be reinitiated by starting the pump or pumps and opening clamps 52, 62, and 64 when the replacement fluids in weighing bag 80 have been replenished to some predetermined reinitiation amount selected to be intermediate between the warning amount and the fail safe amount, preferably at least 30 grams above the fail safe amount. When the weight of fluid in the weighing bag rises above the reinitiation amount, system operations are reinitiated either manually in response to cessation or removal of the fail safe alarm, or automatically by the control means.

This fail safe feature prevents weighing bag 80 from completely emptying so that air never enters flow path 10 from bags 76 or from weighing bag 80. Therefore, once the amount of fluid in bag 80 has been replenished, the system operation can be continued and does not have to be scrapped.

The flow characteristics of each plastic set are necessarily unique due to slight variations in internal diameter, and the cumulative effect of connectors and branch points upon flow dynamics. Thus the performance of pump 72 is different with each flow set used and can change during the course of the procedure. Despite these differences, replacement fluid pump 72 can readily be calibrated to take into account the individual flow characteristics of flow path 10 using the multiple fluid flow isolation, control and alarm system herein. To accomplish this, either before or during plasmapheresis, with clamp means 78 closed, the time required to pump any known amount of fluid from weighing bag 80 through the associated portions of flow path 10 is noted and the rate of delivery of pump 72 is readily derived by known means.

Absolute calibration of pump 72 by this method provides the advantage that the total amount of replacement fluid delivered to the patient from the replacement fluid bags 76 can be determined with great accuracy whether the replacement fluid consists essentially of a single fluid, such as saline, or whether it comprises a mixture of saline and other therapeutic fluids, such as liquid antibiotics and the like. Thus, the physician's instructions regarding the needs of the patient can be accomplished with great accuracy despite the effect of the individual flow characteristics of the harness set upon the performance of pump 72. Preferably the system is recalibrated during each weighing bag cycle during the time the weight in weighing bag 80 moves from the upper working limit to the lower working limit and the pump calibration thus obtained is used to compute the amount of fluid delivered by pump 72 during the next following fill cycle, while the weight in the weighing bag 80 moves between the lower working limit and the upper working limit.

## Claims

1. A method of monitoring fluid flow from a limited fluid source (76) in a container, into a fluid flow system so as to prevent air from the empty container entering the flow system, said method comprising flowing a first isolated amount of fluid from said fluid source (76) into a control container (80), controlling the flow of fluid from the fluid source (76) such that fluid is flowed into the control container (80) when the amount of fluid in the control container reaches a preselected lower working limit amount and fluid flow into the control container is stopped when the amount of fluid in the control container reaches a preselected upper working limit; and flowing fluid from the control container (80) into the flow system,
**characterised in that**:
the control container is a weighing bag (80) and the fluid in the bag is weighed, the lower and upper working limits being fluid weight limits,
a warning alarm is triggered when the weight of fluid in the weighing bag (80) drops below a warning amount, below the lower working limit, and
a replacement fluid source is connected to the weighing bag (80) in response to the warning alarm, so that fluid flows from the replacement fluid source into the weighing bag until the weight of fluid in the weighing bag is raised above the warning amout while fluid continues to flow from the weighing bag (80).

2. A method of Claim 1 including shutting down flow from the weighing bag (80) when the weight of fluid in the bag drops below a fail safe amount, below the warning limit.

3. The method of Claim 2 including the step of stopping fluid flow throughout the fluid flow system in response to a fail safe alarm.

4. The method of Claim 1 or 2, wherein the upper working limit is 200 grams and the lower working limit is 100 grams.

5. The method of any preceding claim wherein the warning amount is from 65 to 85 grams.

6. The method of any preceding claim, wherein the fail safe amount is 15 grams or less.

7. The method of any preceding claim, wherein the fluid flow system is a disposable non-invasive fluid path used for plasmapheresis.

8. The method of any preceding claim, including isolating the weighing bag (80) from flow disturbances during weighing.

9. The method of any preceding claim, including the step of adding together the weights of each isolated amount of fluid flowed into the weighing bag (80) to compute the total amount of fluid flowed therefrom into the flow path.

## Patentansprüche

1. Verfahren zum Überwachen einer Fluidströmung aus einer begrenzten Fluidquelle (76) in einem Behälter in ein Fluidströmungssystem, um zu verhindern, daß Luft aus dem leeren Behälter in das Strömungssystem eintritt, wobei das Verfahren umfaßt, daß eine erste getrennte Fluidmenge aus der Fluidquelle (76) in einen Kontrollbehälter (80) strömt und daß die Fluidströmung aus der Fluidquelle (76) kontrolliert wird, derart, daß das Fluid in den Kontrollbehälter (80) strömt, wenn die Menge des Fluids in dem Kontrollbehälter einen vorgewählten unteren Arbeitsgrenzwert erreicht und daß die Fluidströmung in den Kontrollbehälter gestoppt wird, wenn die Fluidmenge in dem Kontrollbehälter einen vorgewählten oberen Grenzwert erreicht, wobei man das Fluid aus dem Kontrollbehälter (80) in das Strömungssystem strömen läßt,
**dadurch gekennzeichnet,**
**daß** der Kontrollbehälter ein Wägebeutel (80) ist und das Fluid in dem Beutel gewogen wird, wobei die oberen und unteren Arbeitsgrenzwerte Fluidgewichtsgrenzwerte sind, daß ein Warnalarm ausgelöst wird, wenn das Gewicht des Fluids in dem Wägebeutel (80) unter einen Warnwert abfällt, der unterhalb des unteren Arbeitsgrenzwertes liegt,
und **daß** eine Ersatzfluidquelle in Abhängkeit von dem Warnalarm an den Wägebeutel (80) angeschlossen wird, so daß Fluid aus der Ersatzfluidquelle in den Wägebeutel strömt bis das Gewicht des Fluids in dem Wägebeutel oberhalb des Warnwertes angestiegen ist, während das Fluid weiterhin aus dem Wägebeutel (80) strömt.

2. Verfahren nach Anspruch 1,
das den Schritt umfaßt, daß die Strömung aus dem Wägebeutel (80) abgesperrt wird, wenn das Gewicht des Fluids in dem Beutel unter einen ausfallsicheren Wert abfällt, der unterhalb des Warngrenzwertes liegt.

3. Verfahren nach Anspruch 2,
das den Schritt umfaßt, daß in Abhängkeit von einem ausfallsicheren Alarm die Strömung in dem gesamten Fluidströmungssystem gestoppt wird.

4. Verfahren nach Anspruch 1 oder 2,
wobei der obere Arbeitsgrenzwert 200 Gramm beträgt und der untere Arbeitsgrenzwert 100 Gramm beträgt.

5. Verfahren nach einem der vorhergehenden Ansprüche,
wobei der Warnwert 65 Gramm bis 85 Gramm beträgt.

6. Verfahren nach einem der vorhergehenden Ansprüche,
wobei der ausfallsichere Wert 15 Gramm oder weniger beträgt.

7. Verfahren nach einem der vorhergehenden Ansprüche,
wobei das Fluidströmungssystem eine nicht-invasive Einmal-Fluidströmungseinrichtung ist, die für die Plasmapherese verwendet wird.

8. Verfahren nach einem der vorhergehenden Ansprüche,
das den Schritt umfaßt, daß der Wägebeutel (80) während des Wiegens von Strömungsstörungen getrennt wird.

9. Verfahren nach einem der vorhergehenden Ansprüche,
das den Schritt umfaßt, daß die Gewichte von jeder getrennten Menge von Fluid, das in den Wägebeutel (80) strömt, aufaddiert werden, um die Gesamtmenge des Fluids zu berechnen, das von dort in die Strömungsleitung strömt.

## Revendications

1. Procédé de contrôle d'un écoulement de fluide à partir d'une source de fluide limitée (76) dans un conteneur, dans un système d'écoulement de fluide de manière à empêcher que de l'air provenant du conteneur vide ne pénètre dans le système d'écoulement, ledit procédé comprenant la circulation d'une première quantité isolée de fluide à partir de ladite première source (76) dans un conteneur de contrôle (80), le contrôle de l'écoulement de fluide à partir de la source de fluide (76) de sorte que le fluide s'écoule dans le conteneur de contrôle (80) lorsque la quantité de fluide contenue dans le conteneur de contrôle atteint une quantité limite de fonctionnement inférieure présélectionnée et que l'écoulement de fluide dans le conteneur de contrôle soit arrêté lorsque la quantité de fluide dans le conteneur de contrôle atteint une limite de fonctionnement supérieure présélectionnée, et la circulation de fluide à partir du conteneur de contrôle (8) dans le système d'écoulement,
**caractérisé en ce que** :
le conteneur de contrôle est une poche de pesage (80) et le fluide contenu dans la poche est pesé, les limites de fonctionnement inférieure et supérieure étant des limites de poids de fluide,
une alarme d'avertissement est déclenchée lorsque le poids de fluide dans la poche de pesage (80) tombe au-dessous d'une quantité d'avertissement, au-dessous de la limite de fonctionnement inférieure, et
une source de fluide de remplacement est reliée à la poche de pesage (80) en réponse à l'alarme d'avertissement de sorte que le fluide s'écoule à partir de la source de fluide de remplacement dans la poche de pesage jusqu'à ce que le poids de fluide dans la poche de pesage soit élevé au-dessus de la quantité d'avertissement, tandis que le fluide continue de s'écouler à partir de la poche de pesage (80).

2. Procédé selon la revendication 1, comprenant l'arrêt de l'écoulement à partir de la poche de pesage (80) lorsque le poids de fluide dans la poche tombe au-dessous d'une quantité de sécurité, au-dessous de la limite d'avertissement.

3. Procédé selon la revendication 2, comprenant l'étape consistant à arrêter l'écoulement de fluide dans le système d'écoulement de fluide en réponse à une alarme de sécurité.

4. Procédé selon la revendication 1 ou 2, dans lequel la limite de fonctionnement supérieure est de 200 g et la limite de fonctionnement inférieure est de 100 g.

5. Procédé selon l'une quelconque des revendications précédentes, dans lequel la quantité d'avertissement est comprise entre 65 et 85 g.

6. Procédé selon l'une quelconque des revendications précédentes, dans lequel la quantité de sécurité est de 15 g ou moins.

7. Procédé selon l'une quelconque des revendications précédentes, dans lequel le système d'écoulement de fluide consiste en un trajet de fluide non invasif jetable utilisé pour la plasmaphérèse.

8. Procédé selon l'une quelconque des revendications précédentes, comprenant l'isolement de la poche de pesage (80) des perturbations de l'écoulement pendant la pesée.

9. Procédé selon l'une quelconque des revendications précédentes, comprenant l'étape consistant à additionner les poids de chaque quantité isolée de fluide circulant dans la poche de pesage (80) afin de calculer la quantité totale de fluide circulant à partir de celle-ci dans le trajet d'écoulement.
